Europäisches Patentamt

**European Patent Office**

**Office européen des brevets**

(11) Numéro de publication: **0 277 391 B1**

## (12) FASCICULE DE BREVET EUROPEEN

(45) Date de publication de fascicule du brevet: **04.03.92** (51) Int. Cl.⁵: **G01T 1/164**

(21) Numéro de dépôt: **87202638.0**

(22) Date de dépôt: **29.12.87**

Le dossier contient des informations techniques présentées postérieurement au dépôt de la demande et ne figurant pas dans le présent fascicule.

---

(54) **Caméra à scintillation.**

---

(30) Priorité: **06.01.87 FR 8700042**

(43) Date de publication de la demande:
**10.08.88 Bulletin 88/32**

(45) Mention de la délivrance du brevet:
**04.03.92 Bulletin 92/10**

(84) Etats contractants désignés:
**BE DE FR GB IT NL SE**

(56) Documents cités:
**EP-A- 0 166 165**
**FR-A- 2 552 233**
**FR-A- 2 570 507**

(73) Titulaire: **LABORATOIRES D'ELECTRONIOUE PHILIPS**
**3, Avenue Descartes**
**F-94450 Limeil-Brévannes(FR)**
(84) Etats contractants désignés:
**FR**

(73) Titulaire: **N.V. Philips' Gloeilampenfabrieken**
**Groenewoudseweg 1**
**NL-5621 BA Eindhoven(NL)**
(84) Etats contractants désignés:
**BE DE GB IT NL SE**

(72) Inventeur: **Pauzat, Vincent Société Civile S.P.I.D.**
**209, rue de l'Université**
**F-75007 Paris(FR)**
Inventeur: **Jatteau, Michel Société Civile S.P.I.D.**
**209, rue de l'Université**
**F-75007 Paris(FR)**

(74) Mandataire: **Landousy, Christian et al Société Civile S.P.I.D. 156, Boulevard Haussmann**
**F-75008 Paris(FR)**

---

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

**Description**

La présente invention concerne une caméra à scintillation comprenant un cristal scintillateur, une matrice de p photodétecteurs pour la conversion de chaque scintillation en p signaux électriques, p voies d'acquisition prévues pour l'amplification et le filtrage desdits signaux et pour délivrer elles-mêmes p signaux électriques de caractéristiques liées notamment à l'intensité de la scintillation et à la distance de cette scintillation à chacun des photodétecteurs, et un calculateur de sortie destiné à délivrer les coordonnées $x_j$ et $y_j$ d'une scintillation j et l'énergie $E_j$ associée à celle-ci.

Pour déterminer l'image de fixations radioactives à l'intérieur d'un organe, la médecine fait appel, entre autres moyens, au principe de la scintigraphie. Ce principe consiste à introduire dans l'organisme d'un patient un élément radioactif qui va se fixer plus ou moins sur certains organes selon que ceux-ci sont sains ou malades. La mesure de l'intensité de rayonnement gamma émis fournit alors une indication sur la répartition de l'élément radioactif dans l'organisme et constitue donc une aide au diagnostic. Une telle mesure est réalisée à l'aide d'une caméra à scintillation.

Dans les caméras à scintillation traditionnelles, par exemple de type Anger (voir le brevet US-A-3011057), les rayons gamma représentatifs de la distribution radioactive dans le milieu examiné pénètrent, après traversée d'un collimateur, dans un cristal scintillateur. Les scintillations qui se produisent dans ce cristal sont alors détectées par l'intermédiaire de toute une série de tubes photomultiplicateurs (par exemple 19, 37, etc...), après traversée d'un guide de lumière assurant un couplage optique entre le cristal et les tubes. Ces tubes sont répartis devant le bloc optique (cristal + guide de lumière) pour couvrir pratiquement toute sa surface et transformer l'énergie lumineuse de chaque scintillation apparue en un signal électrique mesurable.

A chaque tube photomultiplicateur est alors associée une voie d'acquisition analogique opérant successivement une amplification, une intégration et une mise en forme des signaux fournis par le tube. Les sorties $S_{ij}$ de l'ensemble des voies d'acquisition sont envoyées vers un calculateur qui fournit par estimation les coordonnées $x_j$ et $y_j$ d'une scintillation j et son énergie $E_j$, l'indice i désignant celle des voies d'acquisition qui est concernée. Dans le calculateur, plusieurs types de dispositifs de calcul peuvent être prévus, mais deux d'entre eux essentiellement sont réellement utilisée, à savoir un dispositif de calcul barycentrique à rapport arithmétique, ou un dispositif de calcul barycentrique à rapport logarithmique.

Dans un dispositif de calcul barycentrique à rapport arithmétique, les grandeurs $x_j$, $y_j$, $E_j$ sont données par les expressions :

$$x_j = \frac{X_j}{Z_j} \qquad\qquad (1)$$

$$y_j = \frac{Y_j}{Z_j} \qquad\qquad (2)$$

$$E_j = \sum_{i=1}^{i=p} G_i \, S_{ij} \qquad\qquad (3)$$

Dans ces expressions, on a :

$$X_j = \sum_{i=1}^{i=p} K_i \, S_{ij} \qquad\qquad (4)$$

$$Y_j = \sum_{i=1}^{i=p} H_i \, S_{ij} \qquad\qquad (5)$$

$$Z_j = \sum_{i=1}^{i=p} J_i \, S_{ij} \qquad\qquad (6)$$

les coefficients $G_i$, $K_i$, $H_i$, $J_i$ étant des facteurs de pondération liés à la position de l'axe de chacun des p tubes photomultiplicateurs.

Dans un dispositif de calcul barycentrique à rapport logarithmique, les grandeurs $x_j$, $y_j$, $E_j$ sont cette fois

données par les expressions :

$$x_j = \frac{1}{a} \text{Log} \frac{X_j^+}{X_j^-} \qquad (7)$$

$$y_j = \frac{1}{a} \text{Log} \frac{Y_j^+}{Y_j^-} \qquad (8)$$

$$E_j = \sum_{i=1}^{i=p} G_i \, S_{ij} \qquad (9)$$

avec :

$$X_j^+ = \sum_{i=1}^{i=p} K_i^+ \, S_{ij} \qquad (10)$$

$$X_j^- = \sum_{i=1}^{i=p} K_i^- \, S_{ij} \qquad (11)$$

$$Y_j^+ = \sum_{i=1}^{i=p} H_i^+ \, S_{ij} \qquad (12)$$

$$Y_j^- = \sum_{i=1}^{i=p} H_i^- \, S_{ij} \qquad (13)$$

dans lesquelles les facteurs de pondération sont de façon similaire liés à la position de l'axe de chacun des p tubes photomultiplicateurs.

Dans la suite de la description, on considèrera, sans qu'il s'agisse là d'une limitation de l'invention décrite plus loin, qu'on utilise par exemple un dispositif de calcul barycentrique à rapport arithmétique.

Le transfert des signaux de sortie des p voies d'acquisition vers le calculateur déterminant les coordonnées et l'énergie des événements j est très différent selon que ces signaux sont analogiques, comme dans la plupart des caméras classiques, ou au contraire numériques, comme dans certaines caméras à scintillation de conception plus récente dans lesquelles une conversion analogique-numérique des signaux est effectuée dans les voies d'acquisition.

Lorsque lesdits signaux de sortie sont analogiques, leur transfert s'effectue au moyen de réseaux de résistances, dont la valeur ohmique est proportionnelle aux facteurs de pondération, et d'amplificateurs sommateurs. Un exemple de ce type de transfert de signaux est décrit dans le brevet FR-A- 2.288.987. Selon ce document, la caméra décrite a pour objectif de remédier à l'inconvénient qui consiste à utiliser les signaux de sortie de tous les photodétecteurs pour la localisation des scintillations. A cet effet, ladite caméra comprend des moyens pour sélectionner parmi tous ces signaux de sortie un nombre fixe prédéterminé (trois dans l'exemple proposé) d'entre eux, ceux des photodétecteurs dont les signaux de sortie sont affectés par un rapport signal sur bruit médiocre étant ainsi éliminés. De ce fait, la résolution spatiale de la caméra est améliorée.

Une telle mesure ne permet cependant en aucune manière d'améliorer le taux de comptage maximal de la caméra. Les sociétés demanderesses ont en effet constaté les limitations des réalisations antérieures, en matière de rapidité, notamment lorsque les événements à détecter sont très proches et que les scintillations, également très rapprochées dans le temps, conduisent à un empilement au moins partiel des impulsions électriques qui leur correspondent.

Le but de l'invention est de proposer une caméra à scintillation sensiblement plus rapide que les réalisations antérieures, notamment dans la situation évoquée d'empilement des signaux correspondant aux scintillations.

A cet effet, la caméra selon l'invention est caractérisée en ce que :

(A) lesdites p voies d'acquisition sont prévues pour réaliser l'échantillonnage desdits p signaux, puis la conversion analogique-numérique des échantillons obtenus et la sommation des échantillons correspondant à l'empilement éventuel des signaux individuels associés à plusieurs scintillations rapprochées dans le temps, et délivrer p signaux numériques ;

(B) il est prévu, entre la sortie des p voies d'acquisition et l'entrée du calculateur de sortie, un étage de transfert, prévu pour commander, parmi les p signaux de sortie desdites voies, le transfert vers ledit calculateur d'un nombre aléatoire p' de ces signaux, ce nombre p' étant sélectionné après calcul d'un seuil $m_{oj}$ asservi à l'effet d'empilement des scintillations détectées et à leur énergie globale $E_{mj}$ et élimination des p-p' signaux inférieurs audit seuil asservi, et ledit étage comprenant lui-même :

(a) p soustracteurs recevant d'une part sur une première entrée la sortie correspondante des p voies d'acquisition et d'autre part sur une deuxième entrée commune ledit seuil asservi, évalué par un calculateur de seuil ;

(b) une (p + 1)ième voie d'acquisition fournissant audit calculateur de seuil le signal numérique d'énergie $E_{mi}$ pour l'évaluation du seuil asservi ;

(c) en sortie de ladite (p + 1)ième voie d'acquisition et desdits p soustracteurs, (p + 1) circuits de contrôle de transfert ;

(d) un bus de transfert prévu pour recevoir les (p + 1) signaux de sortie desdits (p + 1) circuits et délivrer vers le calculateur de sortie d'une part le signal d'énergie et d'autre part p' signaux parmi les p signaux de sortie des p premiers circuits de contrôle de transfert ;

(C) ledit calculateur de sortie comprend lui-même :

(a) un étage de sommation numérique recevant les signaux de sortie de l'étage de contrôle de transfert et délivrant principalement les signaux $X_{mj}$, $Y_{mj}$ suivants :

$$X_{mj} = \sum_{i=1}^{i=p'} K_i (M_{ij} - m_{oj})$$

$$Y_{mj} = \sum_{i=1}^{i=p'} H_i (M_{ij} - m_{oj})$$

ainsi que le signal numérique d'énergie $E_{mj}$, les coefficients $K_i$, $H_i$ étant des coefficients de pondération et la grandeur $M_{ij}$ désignant chaque signal fourni par la voie d'acquisition i pour une scintillation j ;

(b) un étage de traitement des événements prévu pour recevoir lesdits signaux $X_{mj}$, $Y_{mj}$, $E_{mj}$ et délivrer pour chaque scintillation j -ou événement j- les trois signaux de coordonnées $x_j$, $y_j$ et d'énergie $E_j$ et comprenant notamment des circuits de calcul de désempilement ;

(D) un étage de détection, séquencement et stockage, comprenant notamment un détecteur de début d'impulsion qui reçoit d'un amplificateur sommateur un signal analogique correspondant à la somme des p signaux de sortie des photodétecteurs et un circuit d'horloge qui engendre les signaux pour la synchronisation, est prévu pour délivrer d'une part les différents signaux d'horloge pour la synchronisation des p voies d'acquisition, de l'étage de contrôle de transfert et du calculateur de sortie, et d'autre part des coefficients de correction destinés à l'étage de traitement des événements.

Dans une variante de réalisation de la caméra ainsi proposée, le calculateur peut également utiliser la grandeur Z pour le calcul des coordonnées. La caméra ainsi proposée est alors caractérisée en ce que l'étage de sommation numérique du calculateur de sortie est prévu pour délivrer également le signal $Z_{mj}$ suivant :

$$Z_{mj} = \sum_{i=1}^{i=p'} J_i (M_{ij} - m_{oj}),$$

le coefficient $J_i$ étant aussi un coefficient de pondération, et en ce que l'étage de traitement des événements est prévu pour recevoir également ledit signal $Z_{mj}$.

Dans l'une ou l'autre de ces structures de caméra, un gain notable en vitesse de transfert de signaux est obtenu, ce qui permet d'atteindre un taux de comptage maximal très notablement supérieur à celui des caméras actuellement les plus performantes.

Le document EP-A-0166165 et le document FR-A-2570507 décrivent bien l'un une caméra à scintillation et l'autre un dispositif de détection de scintillation prenant, en compte l'effet d'empilement éventuel des événements détectés, mais sans associer cette caractéristique à un dispositif de traitement exécutant les diverses opérations prévues dans le cas de la présente caméra et notamment celle qui consiste à ne conserver qu'un nombre aléatoire p' de signaux parmi les p initialement disponibles lors d'une mesure.

D'autres particularités et avantages de l'invention apparaîtront maintenant dans la description qui suit et dans les dessins annexés, donnés à titre d'exemples non limitatifs et dans lesquels :

- les figures 1a à 1c montrent respectivement la forme des signaux individuels correspondant à des scintillations rapprochées entraînant un empilement partiel (figure 1a), la forme du signal global résultant de cet empilement (figure 1b), et la forme du signal représentant pour chaque événement j la valeur mesurée à l'instant $t_{0,j+1}$ et qui résulte de la sommation des échantillons pendant l'intervalle de temps $\theta_{j,j+1}$ pour la voie d'acquisition i (figure 1c) ;
- la figure 2 montre le schéma de principe d'une caméra à scintillation selon l'invention ;
- la figure 3 montre un mode de réalisation de l'étage de contrôle de transfert et du calculateur de la caméra de la figure 2 ;
- les figures 4 et 6 montre deux exemples de réalisation des circuits de calcul de désempilement de l'étage de traitement des événements ;
- la figure 5 montre un exemple de réalisation de l'étage de détection, séquencement et stockage ;
- les figures 7 et 8 montrent deux variantes de réalisation de l'étage de traitement des événements du calculateur représenté sur la figure 3;
- les figures 9 à 11 montrent trois autres variantes de réalisation de l'étage de traitement des événements.

Dans les caméras à scintillation munies de p voies d'acquisition utilisant des circuits convertisseurs-intégrateurs tels que le dispositif décrit dans la demande de brevet français FR-A- 2540995 ou celui décrit dans la demande de brevet français FR-A- 2552233, les signaux obtenus en sortie des voies d'acquisition sont une mesure de la charge des impulsions électriques fournies par les tubes photomultiplicateurs respectifs en répose à une scintillation.

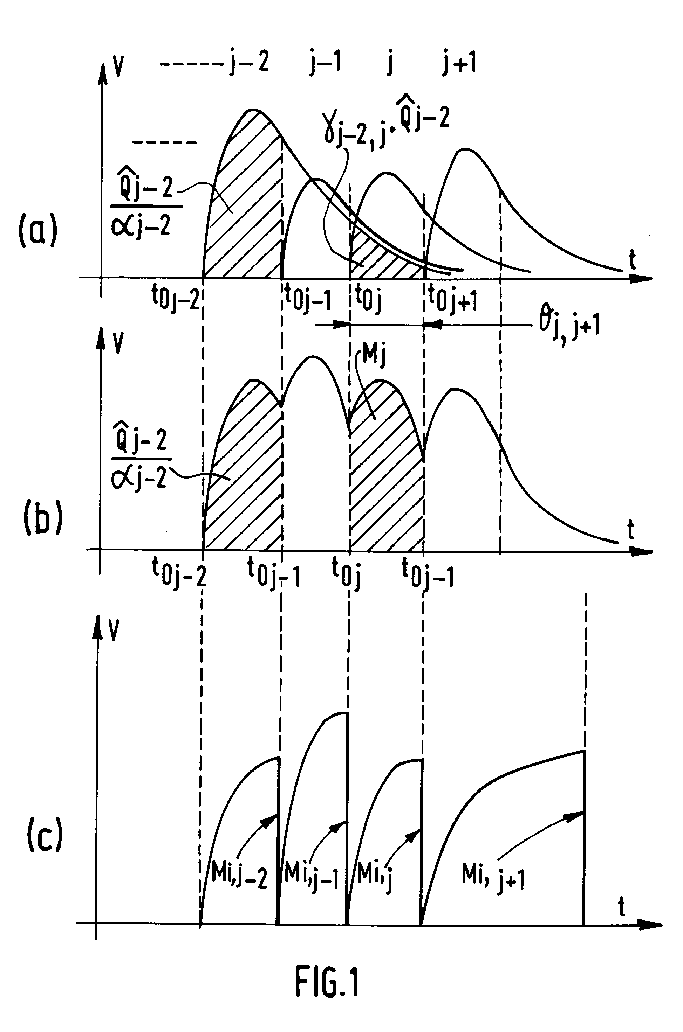

Par exemple, en l'absence d'effet d'empilement, la voie d'acquisition i (i variant de 1 à p) fournirait un signal $S_{ij}$ en réponse à un scintillation j survenant en un point $x_j$, $y_j$ du cristal scintillateur. S'il y a au contraire empilement d'événements comme l'illustre à titre d'exemple la figure 1a qui montre des signaux individuels correspondant à des scintillations rapprochées entraînant un empilement partiel, le signal $M_{ij}$, fourni par la voie d'acquisition i pour un événement j et mesuré à la fin de l'invervalle de temps $\theta_{j,j+1}$ = $t_{0,j+1}$ - $t_{0,j}$ pendant lequel cet événement j n'est perturbé que par les événements précédents, n'est plus $S_{ij}$ mais un signal fonction de $S_{ij}$ et des valeurs $S_{ik}$ (k = j-1, j-2,..., j-q+1, j-q) correspondant aux q événements précédentes qui participent aux perturbations de l'événement j.

Plus précisément, on peut montrer que, pour chacune des voies d'acquisition, le signal mesuré $M_{ij}$ est de la forme théorique suivante :

$$m_{ij} = \frac{S_{ij}}{\alpha_j} + \sum_{k=j-1}^{k=j-q} \gamma_{k,j} S_{ik} \qquad (14)$$

Dans cette relation (14), les coefficients $\alpha_j$ et $\gamma_{kj}$ sont des coefficients de correction, prédéterminés à partir de la connaissance de la forme moyenne, en fonction du temps, des impulsions analogiques fournies à l'entrée des voies d'acquisition. Les coefficients $\alpha_j$, obtenus par extrapolation, sont seulement fonction de l'intervalle de temps $\theta_{j,j+1}$ de la mesure. Ces coefficients $\alpha_j$, supérieurs à 1, tendraient donc vers la valeur 1 si la durée de l'intervalle de mesure $\theta_{j,j+1}$ devenait très grande. Les coefficients $\gamma_{kj}$, obtenus par interpolation, sont fonction de l'intervalle de temps $\theta_{kj}$ séparant chaque événement précédent j-1, j-2, etc... de l'événement j et exprimé par la relation $\theta_{kj}$ = $t_{0,j}-t_{0,k}$ avec k = j-1, j-2,..., q-1, q. Ces coefficients $\gamma_{kj}$, inférieurs à 1, tendraient vers la valeur 0 si la durée des intervalles $\theta_{kj}$ devenait très grande.

Sur la figure 1a, la charge électrique à déterminer, qui serait notée $\hat{Q}_j$ et qui est proportionnelle à la quantité de courant débité pendant la durée $\theta_{j,j+1}$ du signal individuel associé à l'événement j est estimée à partir de la mesure $M_{ij}$ effectuée pendant cette durée et à partir des q charges $\hat{Q}_k$ précédemment estimées.

5

La figure 1b montre le signal somme, ou signal composite, résultant de l'empilement des signaux individuels (de la figure 1a) correspondant à plusieurs scintillations rapprochées dans le temps. La figure 1c montre la forme des signaux $M_{i,j}$ successifs.

Pour l'évaluation des coordonnées $x_j$, $y_j$ de chaque événement j par le calculateur à partir des signaux fournis par les p voies d'acquisition, et ce indépendamment du fait qu'il y ait ou non un empilement d'événements, on introduit un seuil noté $m_{oj}$ et variable d'un événement à l'autre.

De la sorte, la calculateur réalise dans un premier temps les sommes pondérées suivantes :

$$X_{mj} = \sum_{i=1}^{i=p} K_i (M_{ij}-m_{oj}) \qquad (15)$$

$$Y_{mj} = \sum_{i=1}^{i=p} H_i (M_{ij}-m_{oj}) \qquad (16)$$

$$Z_{mj} = \sum_{i=1}^{i=p} J_i (M_{ij}-m_{oj}) \qquad (17)$$

avec $M_{ij} - m_{oj} = 0$ si $M_{ij}$ est inférieur ou égal à $m_{oj}$. Dans ces relations (15) à (17), les facteurs de pondération sont par exemple les mêmes que précédemment.

On peut montrer que la valeur du seuil $m_{oj}$ est avantageusement -sans bien entendu que ce soit le seul choix possible- prise proportionelle à la valeur $E_{mj}$. Le facteur de proportionalité est appelé $f_o$ et est exprimé en V/eV si $m_{oj}$ est une tension et si $E_{mj}$ est exprimé en électronvolts, et l'on a donc :

$$m_{oj} = f_o.E_{mj} \qquad (18)$$

Dans ce type de caméra, le signal numérique $E_{mj}$ résulte de l'échantillonnage, de la numérisation et de l'intégration numérique de la somme pondérée analogique obtenue à la sortie de l'amplificateur sommateur analogique. S'il n'y avait pas d'empilement, cet amplificateur fournirait un signal proportionnel à l'énergie $E_j$ de chacun des événements. Lorsqu'il y a empilement d'événements, $E_{mj}$ s'exprime, conformément à la relation (14), en fonction de l'energie $E_j$ de l'événement j et de celle des q événements précédents, $E_k$, selon la relation suivante :

$$E_{mj} = \frac{E_j}{\alpha_j} + \sum_{k=j-1}^{k=j-q} \gamma_k.E_k \qquad (19)$$

ce qui donne, en remplaçant dans $m_o = f_o.E_{mj}$ :

$$m_{oj} = f_o . (\frac{E_j}{\alpha_j} + \sum_{k=j-1}^{k=j-q} \gamma_{kj}.E_k) \qquad (20)$$

expressions dans lesquelles k varie de j-1, à j-q.

Dans la pratique, la demanderesse a observé que seuls un certain nombre p' de signaux $M_{ij}-m_{oj}$ sont véritablement utiles pour calculer les sommes pondérées $X_{mj}$, $Y_{mj}$, $Z_{mj}$. Cette situation résulte du fait que les tubes photomultiplicateurs situées à grande distance du point de scintillation reçoivent une quantité de lumière très faible, pour laquelle le signal en sortie des voies d'acquisition correspondantes est inférieur au seuil $m_{oj}$. Par grande distance, on entend par exemple une distance de l'ordre de deux fois supérieure à celle qui sépare les axes de deux tubes contigüs.

Le nombre p' de signaux $M_{ij} - m_{oj}$ à transférer, pour chaque scintillation, au calculateur peut donc, en réalité, être inférieur à p. Ce nombre p' dépend essentiellement d'une part de la constitution du bloc optique (cristal + guide de lumière) de la tête de prise de vue de la caméra à scintillation et d'autre part de la valeur du seuil $m_{oj}$ (c'est-à-dire du facteur $f_o$ de proportionelle à l'énergie $E_{mj}$ des événements, facteur qui est préréglé lors des essais de la caméra pour obtenir la meilleure résolution spatiale possible). Le nombre

6

EP 0 277 391 B1

p′ est en pratique par exemple compris entre 9 et 15, compte tenu des fluctuations statistiques, dans des caméras à résolution spatiale élevée, et l'on peut prendre p′ = 12 en moyenne.

On a vu précédemment que la durée de transfert des signaux était égale à p.Δt. Dans une caméra à scintillations comprenant par exemple 61 tubes photomultiplicateurs et donc p = 61 voies d'acquisition, la durée de transfert est égale non plus à 61 Δt mais en moyenne à p′.Δt = 12 Δt dans le cas de cet exemple. Le gain en vitesse de transfert et par suite en taux de comptage maximal est, dans ce cas, de l'ordre de 5. D'une manière générale, ce gain est donné par la valeur du rapport p/p′.

La mise on oeuvre de l'invention est envisageable selon différents modes de réalisation qui vont être successivement décrits, de façon détaillée dans un premier mode de réalisation et seulement dans leurs variantes pour les modes de réalisation suivants.

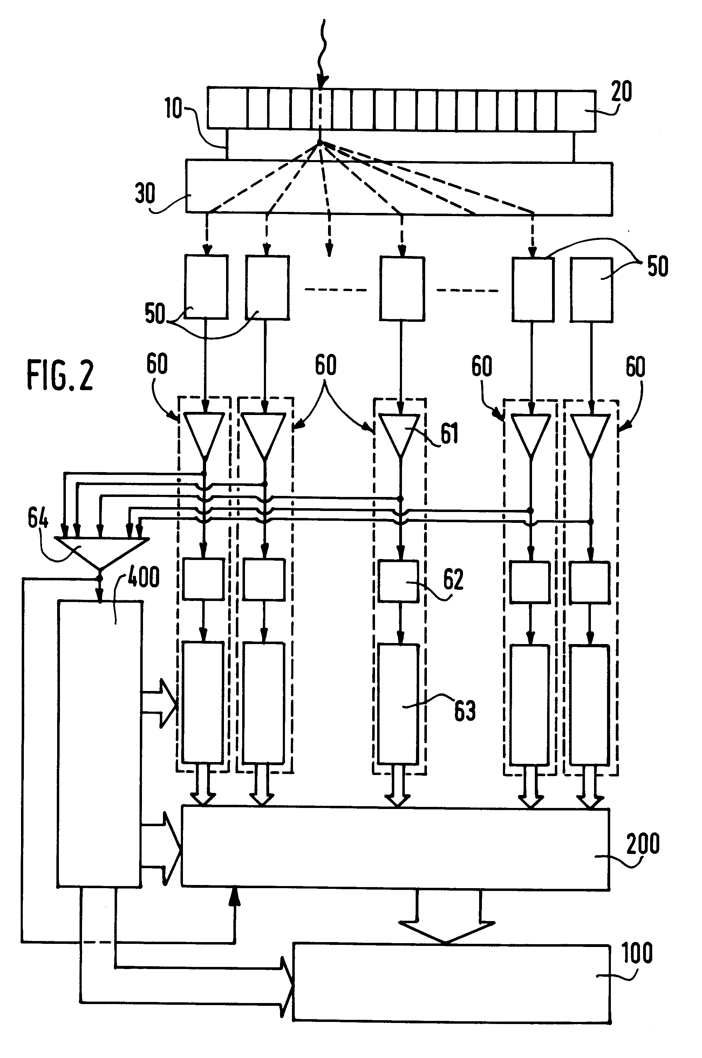

Dans ledit premier mode de réalisation, représenté sur la figure 2, la caméra à scintillation selon l'invention comprend un cristal scintillateur 10 équipé d'un collimateur 20 et destinée à convertir chaque photon reçu en une scintillation. Ce cristal est couplé par l'intermédiaire d'un guide de lumière 30 à la fenêtre d'entrée d'un jeu de p photodétecteurs constitué ici par des tubes photomultiplicateurs 50. Ces tubes 50 convertissent chaque scintillation en un courant qui est alors traité par p voies d'acquisition 60. Ces voies d'acquisition 60 réalisent successivement l'amplification, le filtrage et l'échantillonnage des signaux de sortie des tubes photomultiplicateurs 50 puis la conversion analogique-numérique des échantillons obtenus de la sommation de ces échantillons numériques. La valeur de ces p signaux numériques est liée à celle du courant de sortie des tubes 50 et donc à une fraction de l'intensité de la scintillation initiale, mais différemment selon le taux d'empilement des scintillations (cette fraction est elle-même liée à la réalisation du bloc optique et en particulier à la distance entre le point de scintillation et l'axe des tubes). S'il n'y avait pas d'empilement, la valeur de chacun de ces signaux serait notée $S_{i,j}$ ; l'estimation de ces valeurs en présence d'empilement sera notée $\hat{S}_{i,j}$.

Chacune de ces p voies comprend par exemple en série, pour réaliser les fonctions citées ci-dessus, un circuit 61 d'amplification et de filtrage recevant la sortie du tube 50 correspondant, un circuit de réalignement temporel 62, puis un dispositif de conversion et d'intégration 63 assurant successivement l'échantillonnage des signaux de sortie du circuit 62 correspondant, la conversion analogique-numérique des échantillons obtenus et la sommation de ceux-ci.

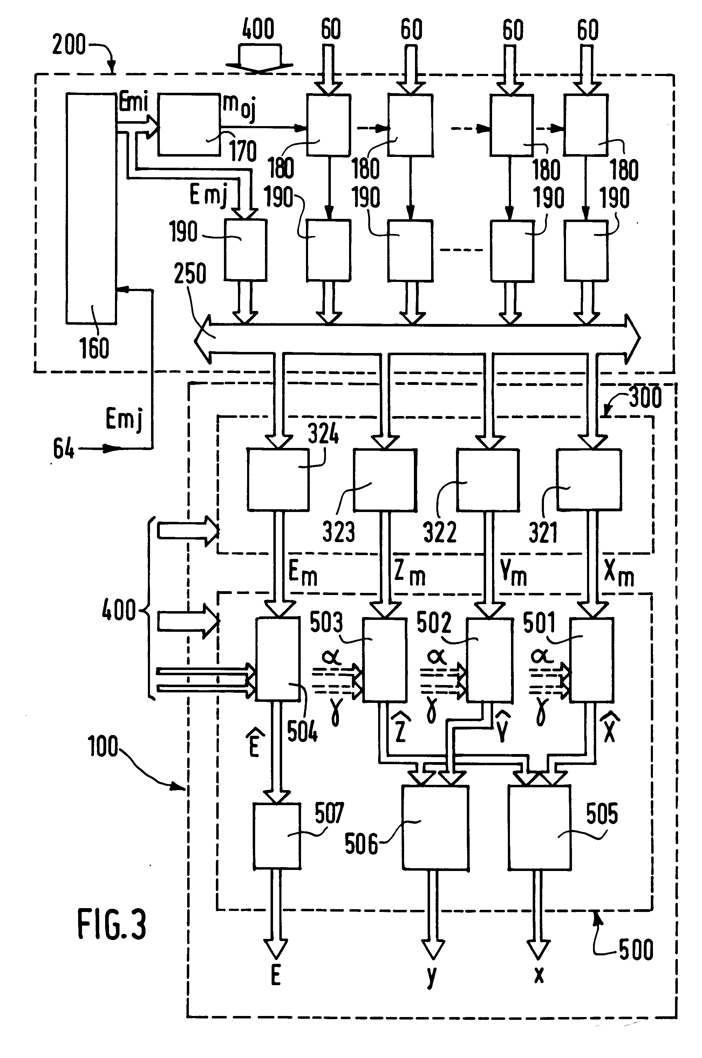

Un amplificateur analogique sommateur 64 reçoit sur p entrées les p sorties analogiques des circuits d'amplification et de filtrage 61 des p voies 60, et la sortie de cet amplificateur sommateur 64 est reliée à l'entrée d'un étage de détection, séquencement et stockage 400 qui sera décrit plus loin. Cette sortie de l'amplificateur 64 est également reliée, comme on peut le voir sur la figure 3, à l'entrée d'une (p + 1)ième voie d'acquisition 160, qui comprend les mêmes éléments 61 à 63 que les p premières voies 60. Cette voie d'acquisition supplémentaire 160 fournit un signal numérique $E_{mj}$ qui est envoyé d'une part vers un calculateur de seuil 170 délivrant sur sa sortie la valeur du seuil asservi $m_{oj}$ et d'autre part vers un circuit de contrôle de transfert 190. Ce seuil $m_{oj}$ est fourni à p soustracteurs de seuil 180 placés en série avec les p voies d'acquisition 60. Ces soustracteurs 180 délivrant respectivement sur leur sortie soit un signal nul si $M_{ij}-m_{oj}$ est inférieur ou égal à 0, soit la valeur $M_{ij}-m_{oj}$ si celle-ci est positive.

Des circuits de contrôle de transfert 190 sont prévus d'une part en série respectivement avec les p soustracteurs 180 et donc avec les p voies d'acquisition 60 correspondantes, et d'autre part en sortie de la voie d'acquisition supplémentaire 160. Ce sont ces circuits de contrôle 190 qui vont permettre, sous la commande de l'étage 400, de ne transférer au calculateur 100 que p′ signaux au lieu de l'ensemble des p signaux disponibles en sortie des p voies d'acquisition 60, ainsi que le signal $E_{mj}$ de sortie de la voie 160, par l'intermédiaire d'un bus de transfert 250.

L'ensemble des éléments 160, 170, 180, 190 et 250 constitué l'étage de contrôle de transfert 200. Il serait bien entendu équivalent, en ce qui concerne la structure de l'invention, que le bus 250 ne soit pas considéré comme inclus dans cet étage 200, mais plutôt dans le calculateur 100 qui est maintenant décrit. Il serait également possible de transférer par une ligne distincte du bus le signal numérique $E_{mj}$ de la sortie de la voie d'acquisition 160 à l'entrée du calculateur 100. Enfin, il est équivalent aussi d'envisager que les p éléments 180 et 190 associés à chacune des p voies d'acquisition soient inclus dans ces voies. La séquence de transfert des données $(M_{ij}-m_{oj})$ sur le bus 250 peut alors être gérée par un protocole connu sous le nom anglais de "Daisy-chain".

Le calculateur 100, représenté sur la figure 3 dans le cas d'un dispositif de calcul barycentrique à rapport arithmétique, est agencé de la façon suivante. Il comprend tout d'abord un étage de sommation numérique 300, composé lui-même de trois dispositifs de sommation pondérée numérique 321, 322, 323, et d'un circuit de réalignement temporel 324 du signal numérique $E_{mj}$. Ces dispositifs réalisent respectivement les sommations pondérées suivantes :

7

$$X_{m,j} = \sum_{i=1}^{i=p'} K_i \, (M_{ij}-m_{oj}) \qquad (21)$$

$$Y_{m,j} = \sum_{i=1}^{i=p'} H_i \, (M_{ij}-m_{oj}) \qquad (22)$$

$$Z_{m,j} = \sum_{i=1}^{i=p'} J_i \, (M_{ij}-m_{oj}) \qquad (23)$$

qui sont analogues aux sommations définies par les relations (15) à (17) mais portent sur seulement p' signaux au lieu de p. Chacun de ces trois dispositifs de sommation pondérée numérique est par exemple du type du multiplieur-accumulateur TDC 1009 commercialisé par la Société TRW, La Jolla, CA 92 038, USA, dont l'une des entrées reçoit la sortie $M_{ij}-m_{oj}$ et dont l'autre reçoit les coefficients de pondération sous forme numérique, stockés dans une mémoire auxiliaire. Dans le cas où l'on utilise effectivement ce type de multiplieur-accumulateur, ladite mémoire auxiliaire, qui doit être synchronisée avec le fonctionnement d'ensemble du calculateur, peut être par exemple incorporée à l'étage de détection, séquencement et stockage 400 décrit plus loin.

Les signaux de sortie $X_{mj}$, $Y_{mj}$, $Z_{mj}$, $E_{mj}$ disponibles en sortie de l'étage de sommation numérique 300 sont alors fournis à un étage de traitement des événements 500. Cet étage 500 comprend, comme indiqué sur la figure 3, quatre circuits de calcul de désempilement 501 à 504, deux diviseurs 505 et 506 et un circuit de réalignement temporel 507.

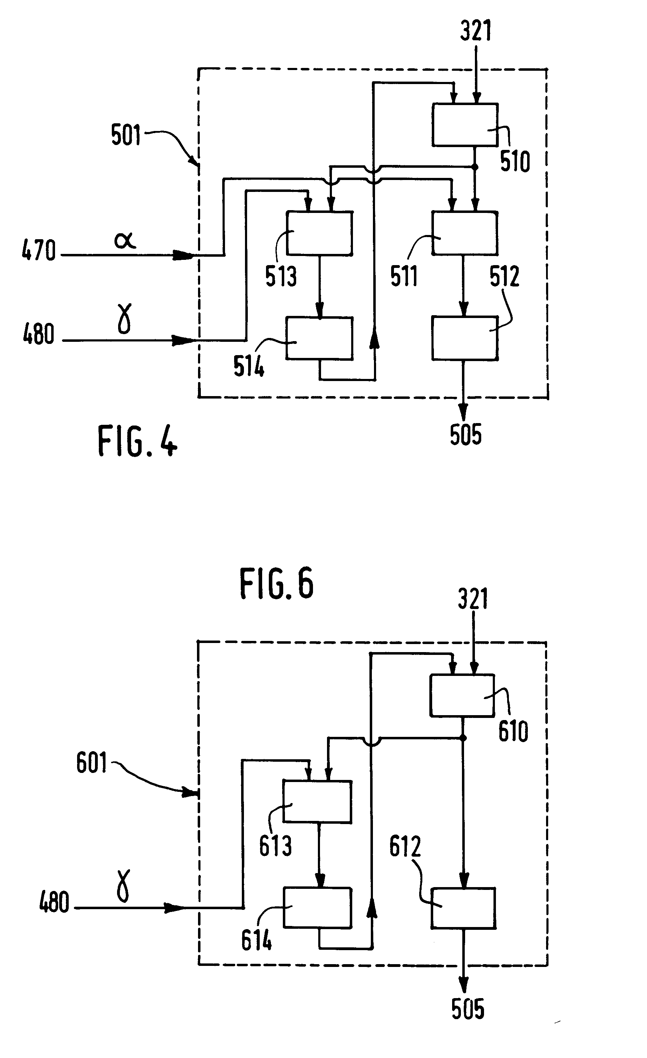

Les quatre circuits 501 à 504 étant identiques, on ne décrit ici que l'un d'eux, par exemple le circuit 501. Celui-ci, représenté sur la figure 4, comprend un soustracteur 510 recevant sur sa première entrée positive la sortie du dispositif de sommation pondérée numérique correspondant 321 (aux dispositifs 321 à 324 correspondent respectivement les circuits 501 à 504). Le soustracteur 510 est suivi d'un premier multiplieur 511 et d'un registre de stockage 512 dont la sortie est celle du circuit 501. Le soustracteur 510 est également suivi, en parallèle sur les éléments 511 et 512, d'un deuxième multiplieur 513 et d'un deuxième registre de stockage 514. Ces multiplieurs peuvent être remplacés par un seul circuit de multiplication associé à un multiplexeur-démultiplexeur temporel. L'entrée négative du soustracteur 510 est reliée à la sortie du registre de stockage 514. la deuxième entrée du multiplieur 511 est reliée à la sortie d'une mémoire 470 stockant les coefficients $\alpha_j$ et celle du multiplieur 513 est reliée à la sortie d'une mémoire 480 stockant les coefficients $\gamma_{j,k}$. Les sorties $\hat{X}$, $\hat{Y}$, $\hat{Z}$, $\hat{E}$ des quatre circuits de calcul de désempilement 501 à 504 sont données, pour l'événement j, par les expressions :

$$\hat{X}_j = \alpha_j \left[ X_{m,j} - \sum_{k=j-1}^{k=j-q} \gamma_{k,j} \, \hat{X}_k \right] \qquad (24)$$

$$\hat{Y}_j = \alpha_j \left[ Y_{m,j} - \sum_{k=j-1}^{k=j-q} \gamma_{k,j} \, \hat{Y}_k \right] \qquad (25)$$

$$\hat{Z}_j = \alpha_j \left[ Z_{m,j} - \sum_{k=j-1}^{k=j-q} \gamma_{k,j} \, \hat{Z}_k \right] \qquad (26)$$

$$\hat{E}_j = \alpha_j \left[ E_{m,j} - \sum_{k=j-1}^{k=j-q} \gamma_{k,j} \, \hat{E}_k \right] \qquad (27)$$

La sortie $\hat{X}$ du circuit de calcul de désempilement 501 est envoyée vers la première entrée du diviseur 505 et la sortie $\hat{Y}$ du circuit 502 vers la première entrée du diviseur 506. La deuxième entrée de chacun de ces diviseurs est constituée par la sortie $\hat{Z}$ du circuit de calcul de désempilement 503. Les trois sorties de l'étage de traitement, qui sont aussi celles du calculateur, sont constituées par la sortie $x_j = \hat{X}_j/\hat{Z}_j$ du diviseur 505, la sortie $y_j = \hat{Y}_j/\hat{Z}_j$ du diviseur 506 et la sortie $\hat{E}_j$ du circuit de réalignement temporel 507 prévu en sortie du circuit de calcul de désempilement 504.

A ces éléments est encore associé l'étage de détection, séquencement et stockage 400. Cet étage 400, représenté sur la figure 5, comprend d'abord un détecteur de début d'impulsion 410, qui reçoit la sortie de l'amplificateur analogique de sommation 64 (voir la figure 2). Ce détecteur 410 est suivi d'un circuit d'horloge 420 qui génère des signaux périodiques assurant la commande d'un circuit de séquencement 450 et celle d'un compteur 430 de ces signaux d'horloge. Le nombre ainsi compté est soumis à un circuit de test 440 dont la sortie est envoyée vers le circuit de séquencement 450. Ce circuit de séquencement 450 assure d'une part la synchronisation des opérations effectuée dans les voies d'acquisition et dans les étages 200, 300 et 500, et valide d'autre part le contenu d'un registre 460 de stockage de la sortie du compteur 430, registre qui est en parallèle sur le circuit de test 440. En sortie du registre 460 sont prévues les deux mémoires 470 et 480 mentionnées plus haut en référence à la figure 4 et stockant respectivement les coefficients $\alpha_j$ et les coefficients $\gamma_{j,k}$.

Dans le premier mode de réalisation qui vient d'être décrit, la structure de caméra proposée a permis de déduire les sommes pondérées exactes $X_j$, $Y_j$, $Z_j$ des valeurs $\hat{X}_{m,j}$, $\hat{Y}_{m,j}$, $\hat{Z}_{m,j}$ et des coefficients $\alpha_j$ et $\gamma_{k,j}$, en utilisant les formules (24) à (27).

Dans un deuxième mode de réalisation de la caméra selon l'invention, la détermination, par le calculateur, des coordonnées des événements peut être effectuée sans introduire d'extrapolation, selon les expressions suivantes :

$$\hat{X}'_j = X_{m,j} - \sum_{k=j-1}^{k=j-q} \gamma_{k,j} \ X'_k \qquad (28)$$

$$\hat{Y}'_j = Y_{m,j} - \sum_{k=j-1}^{k=j-q} \gamma_{k,j} \ Y'_k \qquad (29)$$

$$\hat{Z}'_j = X_{m,j} - \sum_{k=j-1}^{k=j-q} \gamma_{k,j} \ Z'_k \qquad (30)$$

avec toujours $x_j = \hat{X}'_j/\hat{Z}'_j$ et $y_j = \hat{Y}'_j/\hat{Z}'_j$. Dans ce mode de réalisation, l'étage de traitement des événements est maintenant référencé 600, et les trois circuits de calcul de désempilement qui reçoivent respectivement les signaux $X_m$, $Y_m$, $Z_m$ sont, par rapport à la figure 4, modifiés par suppression du multiplieur 511.

Ces trois circuits ont en effet la structure représentée sur la figure 6 pour l'un quelconque, par exemple le premier de ces trois circuits 601 à 603. Ce circuit 601 comprend un soustracteur 610 qui reçoit sur sa première entrée la sortie du dispositif de sommation pondérée numérique correspondant 321. Le soustracteur 610 est suivi d'une part directement d'un registre de stockage 612, dont la sortie est comme précédemment celle du circuit de calcul de désempilement, et d'autre part, en parallèle, d'un multiplieur 613 puis d'un registre de stockage 614. La sortie de ce registre 614 est renvoyée vers la deuxième entrée du soustracteur 610, et l'autre entrée du multiplieur 613 est reliée à la sortie de la mémoire 480 prévue dans l'étage 400 pour le stockage du coefficient $\gamma$.

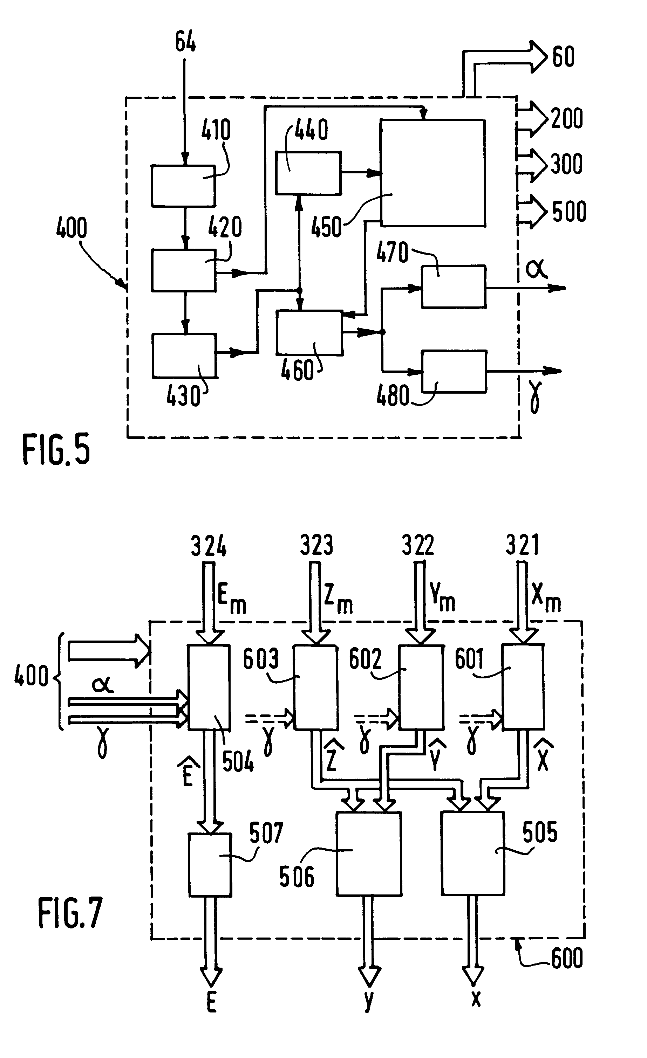

Les deux autres circuits 602 et 603 comprennent des éléments similaires. Le circuit de calcul de désempilement 504 restant inchangé, le calculateur 100 prend maintenant la configuration représentée sur la figure 7. L'énergie E est, comme précédemment, disponibles en sortie du circuit de réalignement temporel 507.

Dans le deuxième mode de réalisation qui vient d'être décrit, la structure du calculateur a permis de procéder au calcul des coordonnées comme précédemment, à partir de sommes pondérées non extrapolées.

Dans un troisième mode de réalisation de la caméra selon l'invention, une autre variante de calculateur est proposée, dans laquelle on détermine d'abord des coordonnées $x_{mj}$, $y_{mj}$, dites mesurées, qui sont entachées d'erreurs du fait de l'empilement des événements :

$x_{mj} = X_{m,j}/Z_{m,j}$     (31)

$y_{mj} = Y_{m,j}/Z_{m,j}$     (32)

A cette détermination succède alors le calcul des coordonnées exactes de l'événement j, au moyen des expressions suivantes :

$$x_j = x_{m,j} - \sum_{k=j-1}^{k=j-q} \Gamma_{k,j} (x_k - x_{mj}) \qquad (33)$$

$$y_j = y_{m,j} - \sum_{k=j-1}^{k=j-q} \Gamma_{k,j} (x_k - x_{mj}) \qquad (34)$$

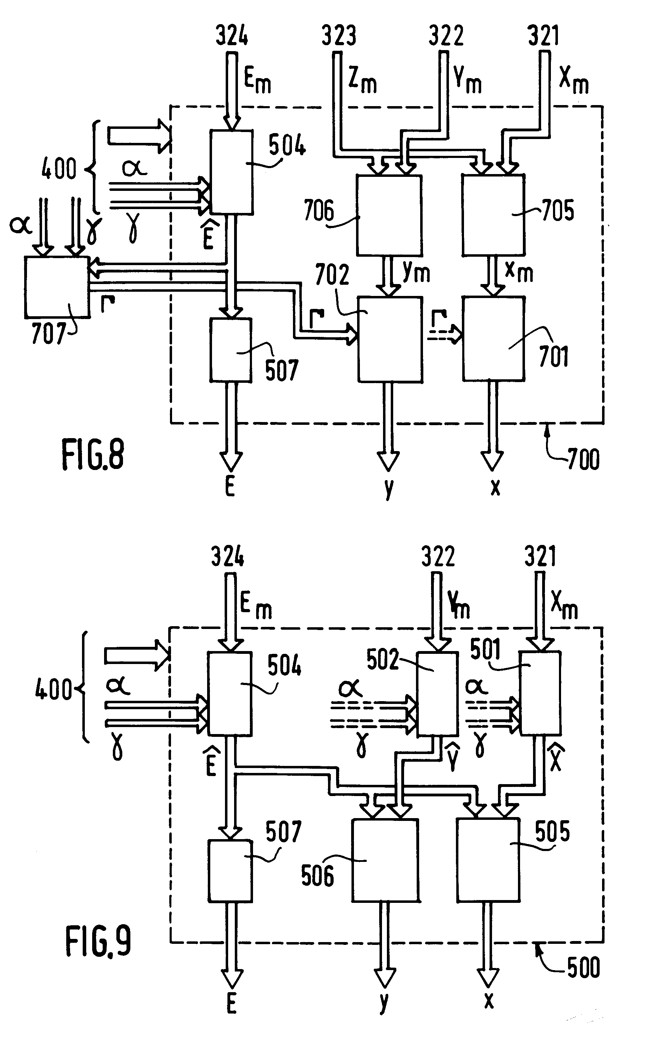

Plus précisément, dans ce troisième mode de réalisation de l'invention, le calculateur 100, représenté cette fois sur la figure 8, comprend un troisième type d'étage de traitement des événements, référencé 700. Dans cette variante de réalisation du calculateur, le traitement de désempilement est effectué non plus sur les signaux $X_m, Y_m, Z_m$ de sortie de l'étage de sommation numérique 300 mais sur les coordonnées $x_{mj}$ et $y_{mj}$ obtenues en sortie de deux diviseurs 705 et 706. Ce diviseur 705 recoit les signaux $X_m$ et $Z_m$ de sortie de l'étage de sommation numérique 300, et le diviser 706 recoit les signaux $Y_m$ et $Z_m$. Les coordonnées $x_j$, $y_j$ correspondant à l'événement j sont alors obtenues en sortie de circuits de calcul de désempilement 701 et 702, à partir d'une part de ces coordonnées dites mesurées et d'autre part des coordonnées déjà connues $x_k$, $y_k$ des événements précédents qui perturbent l'événement j, conformément aux expressions suivantes (33) et (34).

Les coefficients $\Gamma_{k,j}$, qui sont fonctions des intervalles de temps mesurés $\theta_{j,j+1}$, $\theta_{k,j}$ et des rapports $\hat{E}_k/\hat{E}_j$ sont calculés dans un circuit de calcul additionnel 707 qui reçoit d'une part la sortie $\hat{E}$ du circuit de calcul de désempilement 504, toujours présent dans l'étage de traitement, et d'autre part les coefficients $\alpha$ et $\gamma$ délivrés par l'étage de détection, séquencement et stockage 400. Dans cet exemple, le circuit 707 calcule d'une part, à partir des valeurs de $\hat{E}$ successivement reçues, les rapports successifs $\hat{E}_k/\hat{E}_j$ et d'autre part les produits $\alpha_j.\gamma_{k,j}$ à partir desquels sont évalués les coefficients $\Gamma_{k,j}$ selon la relation $\Gamma_{k,j} = \alpha_j.\gamma_{k,j}.\hat{E}_k/\hat{E}_j$. Ces coefficients $\Gamma_{kj}$ sont fournis aux circuits de calcul de désempilement 701 et 702, qui ont une configuration similaire à celle des circuits 601 et 602. L'énergie E est, là encore comme précédemment disponible en sortie du circuit de réalignement temporel 507.

Bien entendu, la présente invention n'est pas limitée aux exemples de réalisation décrits et représentées, à partir desquels d'autres variantes peuvent encore être proposées sans pour cela sortir du cadre de l'invention.

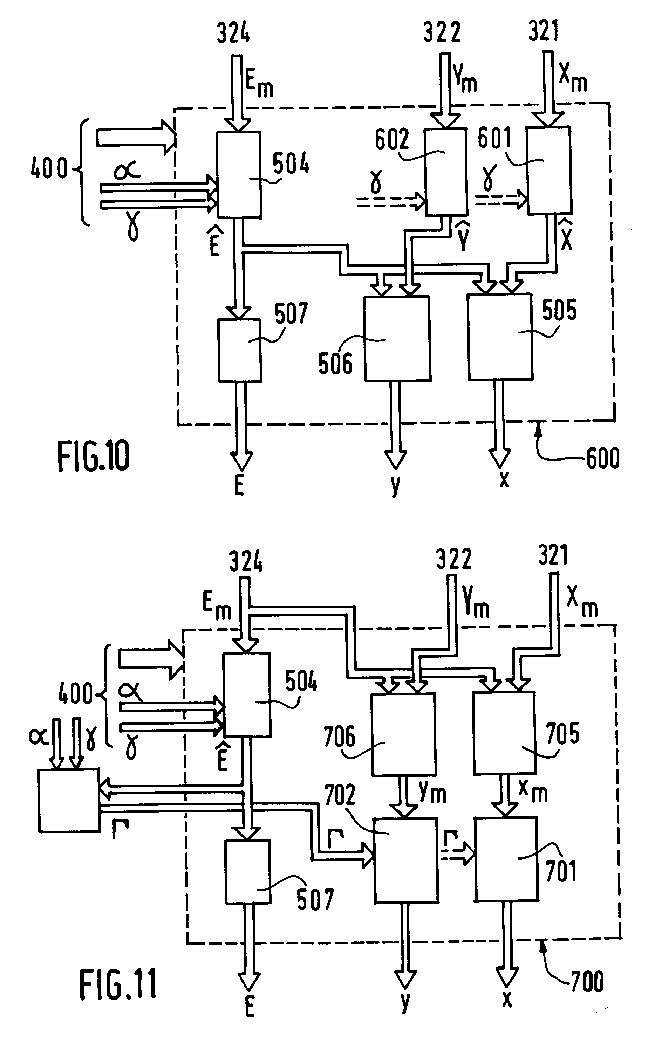

Par exemple, un circuit de réjection en amplitude du nombre des événements à traiter peut être prévu, pour n'effectuer les calculs que sur des événements sélectionnés (à l'aide d'un seuil, d'un fenêtre d'énergie, etc...). Par ailleurs, un multiplexeur temporel peut être prévu pour n'utiliser qu'un diviseur au lieu de deux dans chacun des modes de réalisation des figures 3, 7 et 8. Un circuit de multiplexage temporel peut également être prévu pour réduire le nombre des circuits de calcul de désempilement et n'utiliser qu'un seul circuit de calcul de désempilement au lieu des quatre circuits 501 à 504 dans les cas du mode de réalisation de la figure 3, des quatre circuits 601 à 603 et 504 dans le cas du mode de réalisation de la figure 7, ou des trois circuits 701, 702 et 504 dans le cas du mode de réalisation de la figure 8. L'ensemble des variantes proposées dans ce paragraphe s'applique également aux autres modes de réalisation des figures 9 à 11, mentionnées ci-dessous.

Compte tenu des moyens de correction des défauts de linéarité de d'énergie qui sont mis en oeuvre dans les caméras gamma actuellement disponibles à partir des signaux x, y, E obtenues en sortie du calculateur, on sait en effet qu'il est possible d'utiliser indifféremment Z ou E pour le calcul des coordonnées. Dans ce cas on peut ne calculeur qu'une seule de ces deux grandeurs, la grandeur E, et déduire l'autre, Z, de calculs faisant intervenir des corrections spécifiques de ce choix et exécutées par lesdits moyens.

L'étage de sommation numérique 300 ne comprend plus alors que deux dispositifs de sommation pondérée numérique fournissant des signaux $X_m$, $Y_m$, et de même l'étage de traitement des événements 500 ne comprend plus que trois circuits de calcul de désempilement. Les figures 9 à 11 montrent, en correspondance aux figures 3, 7 et 8 les modifications du calculateur lorsqu'on n'utilise plus que trois voies X, Y, E.

D'autre part, on notera également qu'il est possible, pour travailler à des fréquences plus faibles en régularisant le débit des événements, d'introduire en aval des p voies d'acquisition ou en amont de l'étage de sommation numérique des mémoires FIFO à écriture et lecture commandées par l'étage de détection, séquencement et stockage 400.

Enfin, il est équivalent de considérer que le bus 250 est incorporé au calculateur, dont il est l'élément d'entrée ou d'accès, ou qu'au contraire il lui est associé sans lui être inclus.

**Revendications**

1. Caméra à scintillation comprenant un cristal scintillateur (100), une matrice (50) de p photodétecteurs pour la conversion de chaque scintillation en p signaux électriques, p voies d'acquisition (60) prévues pour l'amplification et le filtrage desdits p signaux et pour délivrer elles-mêmes p signaux électriques de caractéristiques liées notamment à l'intensité de la scintillation et à la distance de cette scintillation à chacun des photodétecteurs, et un calculateur de sortie (100) destiné à délivrer les coordonnées $x_j$ et $y_j$ d'une scintillation j et l'énergie $E_j$ associée à celle-ci, caractérisée en ce que :

    (A) lesdites p voies d'acquisition (60) sont prévues pour réaliser l'échantillonnage desdits p signaux, puis la conversion analogique-numérique des échantillons obtenus et la sommation des échantillons correspondant à l'empilement éventuel des signaux individuels associés à plusieurs scintillations rapprochées dans le temps, et délivrer p signaux numériques ;

    (B) il est prévu, entre la sortie des p voies d'acquisition et l'entrée du calculateur de sortie (100), un étage de contrôle de transfert (200), prévu pour commander, parmi les p signaux de sortie desdites voies (60), le transfert vers ledit calculateur d'un nombre aléatoire p' de ces signaux, ce nombre p' étant sélectionné après calcul d'un seuil $m_{oj}$ asservi à l'effet d'empilement des scintillations détectées et à leur énergie globale $E_{mj}$ et élimination des p-p' signaux inférieurs audit seuil asservi, et ledit étage comprenant lui-même :

    (a) p soustracteurs (180) recevant d'une part sur une première entrée la sortie correspondante des p voies d'acquisition (60) et d'autre part sur une deuxième entrée commune ledit seuil asservi, évalué par un calculateur de seuil ;

    (b) une (p+1)ième voie d'acquisition (160) fournissant audit calculateur de seuil le signal numérique d'énergie $E_{mj}$ pour l'évaluation du seuil asservi ;

    (c) en sortie de ladite (p+1)ième voie d'acquisition (160) et desdits p soustracteurs (180), (p+1) circuits de contrôle de transfert (190) ;

    (d) un bus de transfert (250) prévu pour recevoir les (p+1) signaux de sortie desdits (p+1) circuits (190) et délivrer vers le calculateur de sortie d'une part le signal d'énergie et d'autre part p' signaux parmi les p signaux de sortie des p premiers circuits de contrôle de transfert (190) ;

    (C) ledit calculateur de sortie (100) comprend lui-même :

    (a) un étage de sommation numérique (300) recevant les signaux de sortie de l'étage de contrôle de transfert (200) et délivrant principalement les signaux $X_{mj}, Y_{mj}$ suivants :

$$X_{mj} = \sum_{i=1}^{i=p'} K_i (M_{ij}-m_{oj})$$

$$Y_{mj} = \sum_{i=1}^{i=p'} H_i (M_{ij}-m_{oj})$$

    ainsi que le signal numérique d'énergie $E_{mj}$, les coefficients $K_i$, $H_i$ étant des coefficients de pondération et la grandeur $M_{ij}$ désignant chaque signal fourni par la voie d'acquisition i pour une scintillation j ;

    (b) un étage de traitement des événements (500) prévu pour recevoir lesdits signaux $X_{mj}$, $Y_{mj}$, $E_{mj}$ et délivrer pour chaque scintillation j -ou événement j- les trois signaux de coordonnées $x_j$, $y_j$ et d'énergie $E_j$ et comprenant notamment des circuits de calcul de désempilement ;

    (D) un étage de détection, séquencement et stockage (400), comprenant notamment un détecteur de début d'impulsion (410) qui reçoit d'un amplificateur sommateur (64) un signal analogique correspondant à la somme des p signaux de sortie des photodétecteurs et un circuit d'horloge (420) qui engendre les signaux pour la synchronisation, est prévu pour délivrer d'une part les différents signaux d'horloge pour la synchronisation des p voies d'acquisition (60), de l'étage de contrôle de transfert (200) et du calculateur de sortie (100), et d'autre part des coefficients de correction destinés à l'étage de traitement des événements (500).

2. Caméra à scintillation selon la revendication 1, caractérisée en ce que l'étage de sommation numérique (300) du calculateur de sortie est prévu pour délivrer également le signal $Z_{mj}$ suivant :

$$Z_{mj} = \sum_{i=1}^{i=p'} J_i (M_{ij}-m_{oj}),$$

le coefficient $J_i$ étant aussi un coefficient de pondération, et en ce que l'étage de traitement des événements (500) est prévu pour recevoir également ledit signal $Z_{mj}$.

3. Caméra à scintillation selon la revendication 2, caractérisée en ce que l'étage de traitement des événements (500) comprend quatre circuits de calcul de désempilement (501, 502, 503, 504) ou (601, 602, 603, 504) recevant respectivement les signaux numériques $X_{mj}$, $Y_{mj}$, $Z_{mj}$, $E_{mj}$ et délivrant quatre signaux $\hat{X}$, $\hat{Y}$, $\hat{Z}$, $\hat{E}$, et deux diviseurs (505, 506) délivrant respectivement deux signaux $x_j = \hat{X}/\hat{Z}$ et $y_j = \hat{Y}/\hat{Z}$, les trois signaux $x_j$, $y_j$, $E_j$ étant constitués par les signaux de sortie respectivement du premier diviseur (505), du deuxième diviseur (506) et d'un circuit de réalignement temporel (507) recevant la sortie du quatrième circuit de calcul de désempilement (504), et en ce que les coefficients de correction sont des coefficients de correction par extrapolation et interpolation, $\alpha$ et $\gamma$ respectivement, destinés aux circuits de calcul de désempilement.

4. Caméra à scintillation selon la revendication 3, caractérisée en ce que chacun des circuits de calcul de désempilement comprend un soustracteur (510) recevant sur sa première entrée la sortie correspondante de l'étage de sommation numérique (300), ce soustracteur (510) étant suivi d'une part d'un premier multiplieur (511) et d'un premier registre de stockage (512) et d'autre part, en parallèle sur ce premier multiplieur et ce premier registre, d'un deuxième multiplieur (513) et d'un deuxième registre de stockage (514), la sortie de ce deuxième registre étant reliée à la deuxième entrée du soustracteur (510) et les deuxièmes entrées des premier et deuxième multiplieurs (511,513) étant reliées la première à la sortie d'une mémoire (470) de stockage du coefficient $\alpha$ et la seconde à la sortie d'une mémoire (480) de stockage du coefficient $\gamma$.

5. Caméra à scintillation selon la revendication 3, caractérisée en ce que chacun des circuits de calcul de désempilement comprend un soustracteur (610) recevant sur sa première entrée la sortie correspondante de l'étage de sommation numérique (300), ce soustracteur (610) étant suivi d'une part d'un premier registre de stockage (612) et d'autre part, en parallèle sur ce premier registre, d'un multiplieur (613) et d'un deuxième registre de stockage (614), la sortie de ce deuxième registre étant reliée à la deuxième entrée du soustracteur (610) et la deuxième entrée du multiplieur étant reliée à la sortie d'une mémoire (480) de stockage du coefficient $\gamma$.

6. Caméra à scintillation selon la revendication 2, caractérisée en ce que l'étage de traitement des événements (600) comprend trois circuits de calcul de désempilement (701, 702, 504), deux diviseurs (705, 706), un circuit de réalignement temporel (507), et un circuit de calcul additionnel (707), les deux diviseurs (705, 706) recevant les signaux $X_{mj}$, $Y_{mj}$, $Z_{mj}$ pour délivrer deux signaux $x_{mj} = X_{mj}/Z_{mj}$ et $y_{mj} = Y_{mj}/Z_{mj}$, les premier et deuxième circuits de calcul de désempilement (701,702) recevant lesdits signaux $x_m$, $y_m$ et délivrant les signaux $x_j$,$y_j$ et le troisième circuit de calcul de désempilement (504) recevant le signal $E_{mj}$ et délivrant un signal $\hat{E}$, et le circuit de calcul additionnel (707) recevant d'une part ledit signal $\hat{E}$ et d'autre part lesdits coefficients de correction pour fournir aux premier et deuxième circuits de calcul de désempilement (701, 702) un coefficient de correction additionnel $\Gamma$.

7. Caméra à scintillation selon la revendication 1, caractérisé en ce que l'étage de traitement des événements (500) comprend trois circuits de calcul de désempilement, (501, 502, 504) ou (601, 602, 504), recevant respectivement lesdits signaux numériques $X_{mj}$, $Y_{mj}$, $E_{mj}$ et délivrant trois signaux $\hat{X}$, $\hat{Y}$, $\hat{E}$, et deux diviseurs (505,506) délivrant respectivement deux signaux $x_j = \hat{X}/\hat{E}$ et $y_j = \hat{Y}/\hat{E}$, les trois signaux $x_j$, $y_j$, $E_j$ étant constitués par les signaux de sortie respectivement du premier diviseur (505), du deuxième diviseur (506) et d'un circuit de réalignement temporel (507) recevant la sortie du dernier circuit de calcul de désempilement (504), et en ce que les coefficients de correction sont des coefficients de correction par extrapolation et interpolation, $\alpha$ et $\gamma$ respectivement, destinés aux circuits de calcul de désempilement.

8. Caméra à scintillation selon la revendication 1, caractérisée en ce que l'étage de traitement des événements (600) comprend trois circuits de calcul de désempilement (601, 602, 504), recevant respectivement les signaux numériques $X_{mj}$, $Y_{mj}$, $E_{mj}$ et délivrant trois signaux $\hat{X}$, $\hat{Y}$, $\hat{E}$, et deux diviseurs (505, 506) délivrant respectivement deux signaux $x_j = \hat{X}/\hat{E}$ et $y_j = \hat{Y}/\hat{E}$, les trois signaux $x_j, y_j$, $E_j$ étant constitués par les signaux de sortie respectivement du premier diviseur (505), du deuxième diviseur (506) et d'un circuit de réalignement temporel (507) recevant la sortie du troisième circuit de calcul de désempilement, et en ce que les coefficients de correction sont des coefficients de correction par extrapolation et interpolation, $\alpha$ et $\gamma$ respectivement, destinés auxdits circuits de calcul de désempilement.

9. Caméra à scintillation selon la revendication 1, caractérisée en ce que l'étage de traitement des événements (700) comprend trois circuits de calcul de désempilement (701, 702, 504), deux diviseurs (705,706), un circuit de réalignement temporel (507), et un circuit de calcul additionnel (707), les deux diviseurs (705,706) recevant les signaux $X_{mj}$, $Y_{mj}$, $Z_{mj}$ pour délivrer deux signaux $x_{mj} = X_{mj}/Z_{mj}$ et $y_m = Y_{mj}/Z_{mj}$, les premier et deuxième circuits de calcul de désempilement (701, 702) recevant lesdits signaux $X_m$, $Y_m$ et délivrant les signaux $x_j, y_j$ et le troisième circuit de calcul de désempilement (504) recevant le signal $E_{mj}$ et délivrant un signal $\hat{E}$, et le circuit de calcul additionnel (707) recevant d'une part ledit signal E et d'autre part lesdits coefficients de correction pour fournir aux premier et deuxième circuits de calcul de désempilement (701, 702) un coefficient de correction additionnel $\Gamma$.

10. Caméra à scintillation selon l'une des revendications 4 et 5, caractérisée en ce que les multiplieurs sont remplacés par un seul circuit de multiplication associé à un multiplexeur-démultiplexeur temporel.

11. Caméra à scintillation selon l'une des revendications 3 à 9, caractérisée en ce que les diviseurs sont remplacés par un seul circuit de division associé à un multiplexeur-démultiplexeur temporel.

12. Caméra à scintillation selon l'une des revendications 1 à 11, caractérisée en ce que les circuits de calcul de désempilement sont remplacés par un seul de ces circuits auquel est associé un multiplexeur-démultiplexeur temporel.

13. Caméra à scintillation selon l'une des revendications 1 à 12, caractérisée en ce qu'est prévu un circuit de réjection en amplitude pour la réduction du nombre d'événements à traiter.

14. Caméra à scintillation selon l'une des revendications 1 à 13, caractérisée en ce que des mémoires dites FIFO sont prévues en aval des p voies d'acquisition ou en amont de l'étage de sommation numérique (300).

**Claims**

1. A scintillation camera, including a scintillation crystal (100), a matrix (50) of p photodetectors for converting each scintillation into p electrical signals, p acquisition channels (60) for amplifying and filtering said signals and for delivering p electrical signals of characteristics linked in particular with the intensity of the scintillation and with the distance of this scintillation from each of the photo-detectors, and an output computer (100) intended to deliver the coordinates $x_j$ and $y_j$ of a scintillation j and the energy $E_j$ associated therewith, characterized in that:

(A) said p acquisition channels (60) are provided to sample said p signals, followed by analog-digital conversion of the samples obtained and summing of the samples corresponding to the pile-up, if any, of the individual signals associated with several scintillations which are close in time, and to deliver p digital signals;

(B) there is provided, between the output of the p acquisition channels and the input of the output computer (100), a transfer control stage (200) which is intended to control, from among the p output signals of said channels (60), the transfer to said computer of a random number p' of these signals, this number p' being selected after computation of a threshold $M_{oj}$ dependent on the pile-up effect of the detected scintillations and on their overall energy $E_{mj}$, and to eliminate the p-p' signals lower than said dependent threshold, said stage itself comprising:

(a) p subtractors (180) receiving on the one hand on a first input the corresponding output of the p acquisition channels (60) and, on the other hand, on a second common input said dependent threshold, evaluated by a threshold computer;

(b) a $(p+1)^{th}$ acquisition channel (160) supplying said threshold computer with the digital energy signal $E_{m,j}$ for the evaluation of the dependent threshold;

(c) connected to the output of said $(p+1)^{th}$ acquisition channel (160) and of said p subtractors (180), (p + 1) transfer control circuits (190);

(d) a transfer bus (250) for receiving the (p + 1) output signals of said (p + 1) circuits (190) and for delivering to the output computer on the one hand the energy signal and on the other hand p' signals from among the p output signals of the p first transfer control circuits (190);

(C) said output computer (100) itself includes:

(a) a digital summing stage (300) receiving the output signals from the transfer control stage (200) and delivering mainly the following signals $X_{mj}$, $Y_{mj}$:

$$X_{m,j} = \sum_{i=1}^{i=p'} K_i \ (M_{ij} - m_{oj})$$

$$Y_{m,j} = \sum_{i=1}^{i=p'} H_i \ (M_{ij} - m_{oj})$$

as well as the digital energy signal $E_{mj}$, the coefficients $K_i$, $H_i$, being weighting coefficients, and the quantity $M_{ij}$ designating any signal supplied by the acquisition channel i for a scintillation j;

(b) an event processing stage (500) for receiving said signals $X_{mj}$, $Y_{mj}$, $E_{mj}$ and for delivering, for each scintillation j, or event j, the three signals of the coordinates $x_j$, $y_j$ and the enerrgy $E_j$, and comprising notably unpiling computation circuits;

(D) a detection, sequencing and storage stage (400), comprising notably a pulse start detector (410) which receives from a summing amplifier (64) an analog signal which corresponds to the sum of the p output signals of the photodetectors and a clock circuit which generates the signals for the synchronization, is provided for delivering on the one hand the various clock signals for the synchronization of the p acquisition channels (60), the transfer control stage (200), and the output computer (100) and, on the other hand, correction coefficients intended for the event processing stage (500).

2. A scintillation camera as claimed in Claim 1, characterized in that the digital summing stage (300) of the output computer is provided to deliver also the following signal $Z_{mj}$:

$$Z_{mj} = \sum_{i=1}^{i=p'} J_i \ (M_{ij} - m_{oj}) \ ,$$

the coefficient $J_i$ also being a weighting coefficient, and in that the event processing stage (500) is provided to receive also said signal $Z_{mj}$.

3. A scintillation camera as claimed in Claim 2, characterized in that the event processing stage (500) includes four unpiling computation circuits (501, 502, 503, 504) or (601, 602, 603, 504), respectively receiving the digital signals $X_{mj}$, $Y_{mj}$, $Z_{mj}$, $E_{mj}$ and delivering four signals $\widehat{X}$, $\widehat{Y}$, $\widehat{Z}$, $\widehat{E}$, and two dividers (505, 506), respectively delivering two signals $x_j = \widehat{X}/\widehat{Z}$ and $y_j = \widehat{Y}/\widehat{Z}$, the three signals $x_j$, $y_j$, $E_j$ being respectively constituted by the output signals of the first divider (505), the second divider (506) and a time realignment circuit (507) receiving the output of the fourth unpiling computation circuit (504), and in that the correction coefficients are coefficients for correction by extrapolation and interpolation, $\alpha$ and $\gamma$, respectively, intended for the unpiling computation circuits.

4. A scintilltion camera as claimed in Claim 3, characterized in that each of the unpiling computation circuits includes a subtractor (510) receiving on its first input the corresponding output of the digital summation stage (300), which subtractor (510) is followed on the one hand by a first multiplier (511) and a first storage register (512) and, on the other hand, in parallel with said first multiplier and said first register , a second multiplier (513) and a second storage register (514), the output of this second register being connected to the second input of the subtractor (510), the second inputs of the first and the second multiplier (511, 513) being respectively connected to the output of a memory (470) for storing the coefficient $\alpha$ and to the output of a memory (480) for storing the coefficient $\gamma$.

5. A scintillation camera as claimed in Claim 3, characterized in that each of the unpiling computation circuits includes a subtractor (610) receiving on its first input the corresponding output of the digital summation stage (300), this subtractor (610) being followed on the one hand by a first storage register (612) and, on the other hand, in parallel with said first register, by a first multiplier (613) and a second storage register (614), the output of said second register being connected to the second input of the subtractor (610) and the second input of the multiplier being connected to the output of a memory (480) for storing the coefficient $\gamma$.

6. A scintillation camera as claimed in Claim 2, characterized in that the event processing stage (600) includes three unpiling computation circuits (701, 702, 504), two dividers (705, 706), a time realignment circuit (507), and an additional computation circuit (707), the two dividers (705, 706) receiving the signals $X_{mj}$, $Y_{mj}$, $Z_{mj}$ in order to deliver two signals $x_{mj} = X_{mj}/Z_{mj}$ and $y_{mj} = Y_{mj}/Z_{mj}$, the first and second unpiling computation circuits (701, 702) receiving said signals $x_m$, $y_m$ and delivering the signals $x_j$, $y_j$ and the third unpiling computation circuit (504) receiving the signal $E_{mj}$ and delivering a signal $\hat{E}$ ,the additional computatin circuit (707) receiving on the one hand said signal $\hat{E}$ and on the other hand said correction coefficients in order to supply the first and the second unpiling computation circuit (701, 702) with an additional correction coefficient $\Gamma$.

7. A scintillation camera as claimed in Claim 1, characterized in that the event processing stage (500) includes three unpiling computation circuits (501, 502, 504) or (601, 602, 504), respectively delivering said digital signals $X_{mj}$, $Y_{mj}$, $E_{mj}$ and delivering three signals $\hat{X}$ , $\hat{Y}$ , $\hat{E}$ , and two dividers (505, 506), respectively delivering two signals $x_j = \hat{X}/\hat{E}$ and $y_j = \hat{Y}/\hat{E}$ ,the three signals $x_j$, $y_j$, $E_j$ being constituted respectively by the output signals of the first divider (505), the second divider (506) and a time realignment circuit (507) receiving the output of the last unpiling computation circuit (504), and in that the correction coefficients are coefficients for correction by extrapolation and interpolation, $\alpha$ and $\gamma$ respectively, for the unpiling computation circuits.

8. A scintillation camera as claimed in Claim 1, characterized in that the event processing stage (600) comprises three unpiling computation circuits (601, 602, 504), respectively receiving the digital signals $X_{mj}$, $Y_{mj}$, $E_{mj}$ and delivering three signals $\hat{X}$ , $\hat{Y}$ , $\hat{E}$ , and two dividers (505, 506), respectively delivering two signals $x_j = \hat{X}/\hat{E}$ and $y_j = \hat{Y}/\hat{E}$ , the three signals $x_j$, $Y_j$, $E_j$ being constituted respectively by the output signals of the first divider (505), the second divider (506) and a time realignment circuit (507) receiving the output of the third unpiling computatin circuit, and in that the correction coefficients are coefficients for correction by extrapolation and interpolation, $\alpha$ and $\gamma$ resepctively, for said unpiling computation circuits.

9. A scintillation camera as claimed in Claim 1, characterized in that the event processing stage (700) includes three unpiling computation circuits (701, 702, 504), two dividers (705, 706), a time realignment circuit (507) and an additional computation circuit (707), the two dividers (705, 706) receiving the signals $X_{mj}$, $Y_{mj}$, $Z_{mj}$ in order to deliver two signals $x_{mj} = X_{mj}/Z_{mj}$ and $y_{mj} = Y_{mj}/Z_{mj}$, the first and second unpiling computation circuits (701, 702) receiving said signals $x_m$, $y_m$ and delivering the signals $X_j$, $Y_j$, and the third unpiling computation circuit circuit (504) receiving the signal $E_{mj}$ and delivering a signals $\hat{E}$ , the additional computation circuit (707) receiving on the one hand said signal E and on the other hand said correction coefficients in order to supply the first and the second unpiling computation circuit (701, 702) with an additional correction coefficient $\Gamma$.

10. A scintillation camera as claimed in one of the Claims 4 and 5, characterized in that the multipliers are replaced by a single multiplication circuit associated with a time multiplexer-demultiplexer.

11. A scintillation camera as claimed in any one of the Claims 3 to 9, characterized in that the dividers are replaced by a single division circuit associated with a time multiplexer-demultiplexer.

12. A scintillation camera as claimed in any of the Claims 1 to 11, characterized in that the unpiling computation circuits are replaced by a single one of these circuits with which there is associated a time multiplexer-demultiplexer.

**13.** A scintillation camera as claimed in any one of the Claims 1 to 12, characterized in that there is provided an amplitude rejection circuit for the reduction of the number of events to be processed.

**14.** A scintillation camera as claimed in any one of the Claims 1 to 13, characterized in that so-called FIFO memories are provided downstream of the p acquisition channels or upstream of the digital summing stage (300).

**Patentansprüche**

**1.** Szintillationskamera mit einem Szintiilatorkristall (100), einer Matrix (50) aus p Photodetektoren zum Umsetzen jeder Szintillation in p elektrische Signale, mit p Erfassungswegen (60) zum Verstärken und Filtern der p Signale und zum anschließenden Ausgeben von p elektrischen Signalen, deren Eigenschaften sich insbesondere auf die Szintillationsintensität und auf den Abstand zwischen dieser Szintillation zu jedem der Photodetektoren beziehen, und mit einem Ausgangsrechner (100) zum Ausgeben der Koordinaten $x_j$ und $y_j$ einer Szintillation j und der szintillationsbedingten Energie Ej, dadurch gekennzeichnet, daß

(A) die p Erfassungswege (60) zum Ausführen der Abtastung der p Signale und danach zum analog/digitalen Wandeln der erhaltenen Proben und der Summierung der Proben entsprechend einer möglichen Stapelung der einzelnen, verschiedenen zeitlich benachbarten Szintillationen zugeordneten Signale und zum Ausgeben von p digitalen Signalen dienen,

(B) zwischen dem Ausgang der p Erfassungswege und dem Eingang des Ausgangsrechners (100) eine Übertragungssteuerstufe (200) zum Regeln der Übertragung einer beliebigen Anzahl p' der Ausgangssignale der Wege (60) aus den p Ausgangssignalen auf den Rechner vorgesehen ist, wobei diese Anzahl p' nach der Errechnung einer von der Auswirkung der Stapelung der detektierten Szintillationen und ihrer Gesamtenergie $E_{mj}$ und der Entfernung der unter dieser abhängigen Schwelle liegenden p-p' Signale abhängigen Schwelle $m_{oj}$ gewählt wird, und diese Stufe selbst folgende Elemente enthalt:

(a) p Subtrahierer (180), die einerseits an einem ersten Eingang die entsprechenden Ausgangssignale der p Erfassungswege (60) und andererseits an einem zweiten gemeinsamen Eingang die abhängige, von einem Schwellenrechner errechnete Schwelle empfangen,

(b) einen (p + 1)-ten Erfassungsweg (160), der dem Schwellenrechner das digitale Energiesignal $E_{mj}$ zum Auswerten der abhängigen Schwelle zuleitet,

(c) am Ausgang des (p + 1)-ten Erfassungswegs (160) und der p Subtrahierer (180) (p + 1) Übertragungssteuerschaltungen (190),

(d) einen Übertragungsbus (250) zum Empfangen der (p + 1) Ausgangssignale der (p + 1) Schaltungen (190) und zum Ausgeben einerseits des Energiesignals und andererseits von p' Signalen aus den p Ausgangssignalen der p ersten Übertragungssteuerschaltungen (190) nach dem Ausgangsrechner,

(C) der Ausgangsrechner (100) selbst folgende Elemente enthält:

(a) eine digitale Summierstufe (300), die die Ausgangssignale der Übertragungssteuersstufe (200) empfängt und im wesentlichen die Signale $X_{mj}$, $Y_{mj}$, wie folgt:

$$X_{mj} = \sum_{i=1}^{i=p'} K_i \ (M_{ij}-m_{oj}) \qquad Y_{mj} = \sum_{i=1}^{i=p'} H_i \ (M_{ij}-m_{oj})$$

sowie das digitale Energiesignal $E_{mj}$ ausgibt, wobei die Koeffizienten $K_i$ und $H_i$ Gewichtungskoeffizienten sind und die Größe $M_{ij}$ jedes über den Erfassungsweg i für eine Szintillation j gelieferte Signal darstellt,

(b) eine Ereignisbearbeitungsstufe (500) zum Empfangen der Signale $X_{mj}$, $Y_{mj}$, und $E_{mj}$ und zum Ausgeben der drei Koordinaten- und Energiesignale $x_j$, $y_j$ und $E_j$, für jede Szintillation j - oder Ereignis j -, welche Stufe insbesondere die Entstapelungsberechnungsschaltungen enthält,

(D) eine Detektor-, Sortierer- und Speicherstufe (400) insbesondere mit einem Impulsbeginndetektor (410), der aus einem Summierverstärker (64) ein analoges Signal entsprechend der Summe der p Ausgangssignale der Photodetektoren empfängt, und wobei eine Taktschaltung (420), die die Signale für die Synchronisation erzeugt, zum Ausgeben einerseits der verschiedenen Taktsignale für die Synchronisation der p Erfassungswege (60), der Übertragungssteuerstufe (200) und des Ausgangsrechners (100) und andererseits der Korrekturkoeffizienten zur Ereignisbearbeitungsstufe (500) vorgesehen ist.

2. Szintillationskamera nach Anspruch 1,
dadurch gekennzeichnet, daß die digitale Summierstufe (300) des Ausgangsrechners ebenfalls zum Ausgeben des folgenden Signals $Z_{mj}$ vorgesehen ist:

$$Z_{mj} = \sum_{i=1}^{i=p'} J_i \, (M_{ij} - m_{oj}) \, ,$$

wobei der Koeffizient $J_i$ ebenfalls ein Gewichtungskoeffizient ist, und daß die Ereignisbearbeitungsstufe (500) ebenfalls zum Empfangen des Signals $Z_{mj}$ vorgesehen ist.

3. Szintiliationskamera nach Anspruch 2,
dadurch gekennzeichnet, daß die Ereignisbearbeitungsstufe (500) vier Entstapelungsberechnungsschaltungen (501, 502, 503, 504) oder (601, 602, 603, 504), die die Digitalsignale $X_{mj}$. $Y_{mj}$, $Z_{mj}$ bzw. $E_{mj}$ empfangen und vier Signale $\hat{X}$, $\hat{Y}$, $\hat{Z}$, $\hat{E}$ ausgeben, und zwei Teiler (505, 506) enthält, die zwei Signale $x_j = \hat{X}/\hat{Z}$ bzw. $y_j = \hat{Y}/\hat{Z}$ ausgeben, wobei die drei Signale $x_j$, $y_j$, $E_j$ durch die Ausgangssignale des ersten Teilers (505), des zweiten Teilers (506) bzw. einer Schaltung (507) zum zeitlichen Wiederausrichten gebildet werden, die das Ausgangssignal der vierten Entstapelungsberechnungsschaltung (504) empfängt, und daß die Korrekturkoeffizienten Koeffizienten zur Korrektur mittels Extrapolation und Interpolation $\alpha$ bzw. $\gamma$ für die Entstapelungsberechnungsschaltungen sind.

4. Szintillationskamera nach Anspruch 3,
dadurch gekennzeichnet, daß jede der Entstapelungsberechnungsschaltungen einen Subtrahierer (510) enthält, der auf seinem ersten Eingang das entsprechende Ausgangssignal der digitalen Summierstufe (300) empfängt, wobei diesem Subtrahierer (510) einerseits ein erster Vervielfacher (511) und ein erstes Speicherregister (512) und andererseits in Parallelschaltung mit diesem ersten Vervielfacher und diesem ersten Register ein zweiter Vervielfacher (513) und ein zweites Speicherregister (514) folgen, wobei der Ausgang dieses zweiten Registers mit dem zweiten Eingang des Subtrahierers (510) verbunden ist und von den zweiten Eingängen der ersten und zweiten Vervielfacher (511, 513) der erste mit dem Ausgang eines Koeffizient-$\alpha$– Speichers (470) und der zweite mit dem Ausgang eines Koeffizient-$\gamma$-Speichers (480) verbunden sind.

5. Szintillationskamera nach Anspruch 3,
dadurch gekennzeichnet, daß jede der Entstapelungsberechnungsschaltungen einen Subtrahierer (610) enthält, der auf seinem ersten Eingang das entsprechende Ausgangssignal der digitalen Summierstufe (300) empfängt, wobei diesem Subtrahierer (610) einerseits ein erstes Speicherregister (612) und andererseits in Parallelschaltung mit diesem ersten Register ein Vervielfacher (613) und ein zweites Speicherregister (614) folgen, wobei der Ausgang dieses zweiten Registers mit dem zweiten Eingang des Subtrahierers (610) verbunden ist und der zweite Eingang des Vervielfachers mit dem Ausgang eines Koefflzient-$\gamma$-Speichers (480) verbunden ist.

6. Szintillationskamera nach Anspruch 2,
dadurch gekennzeichnet, daß die Ereignisbearbeitungsstufe (600) drei Entstapelungsberechnungsschaltungen (701, 702, 704), zwei Teiler (705, 706), eine Schaltung (507) zum zeitlichen Wiederausrichten und eine zusätzliche Rechenschaltung (707) enthält, wobei die zwei Teiler (705, 706) die Signale $X_{mj}$. $Y_{mj}$, $Z_{mj}$ zum Ausgeben von zwei Signalen $x_{mj} = X_{mj}/Z_{mj}$ und $y_{mj} = Y_{mj}/Z_{mj}$ empfangen, wobei die ersten und zweiten Entstapelungsberechnungsschaltungen (701, 702) die Signale $x_m$, $y_m$ empfangen und die Signale $x_j$, $y_j$ ausgeben, und die dritte Entstapelungsberechnungsschaltung (504) das Signal $E_{mj}$ empfängt und ein Signal $\hat{E}$ ausgibt, und daß die zusätzliche Berechnungsschaltung (707) einerseits das Signal $\hat{E}$ und andererseits die Korrekturkoeffizienten zum Liefern eines zusätzlichen Korrekturkoeffizienten $\Gamma$ an die ersten und zweiten Entstapelungsberechnungsschaltungen (701, 702) empfängt.

7. Szintiilationskamera nach Anspruch 1,

dadurch gekennzeichnet, daß die Ereignisbearbeitungsstufe (500) drei Entstapelungsberechnungsschaltungen (501, 502, 504) oder (601, 602, 504), die die Digitalsignale $X_{mj}$, $Y_{mj}$, bzw. $E_{mj}$ empfangen und drei Signale $\hat{X}$, $\hat{Y}$, $\hat{E}$ ausgeben, und zwei Teiler (505, 506) enthält, die zwei Signale $x_j = \hat{X}/\hat{E}$ bzw. $y_j = \hat{Y}/\hat{E}$ ausgeben, wobei die drei Signale $x_j$, $y_j$, $E_j$ durch die Ausgangssignale des ersten Teilers (505), des zweiten Teilers (506) bzw. einer Schaltung (507) zum zeitlichen Wiederausrichten gebildet werden, die das Ausgangssignal der letzten Entstapelungsberechnungsschaltung (504) empfängt, und daß die Korrekturkoeffizienten Koeffizienten zur Korrektur mittels Extrapolation und Interpolation $\alpha$ bzw. $\gamma$ für die Entstapelungsberechnungsschaltungen sind.

8. Szintillationskamera nach Anspruch 1,
dadurch gekennzeichnet, daß die Ereignisbearbeitungsstufe (600) drei Entstapelungsberechnungsschaltungen (601, 602, 504), die die Digitalsignale $X_{mj}$, $Y_{mj}$, bzw. $E_{mj}$ empfangen und drei Signale $\hat{X}$, $\hat{Y}$, $\hat{E}$ ausgeben, und zwei Teiler (505, 506) enthält, die zwei Signale $x_j = \hat{X}/\hat{E}$ bzw. $y_j = \hat{Y}/\hat{E}$ ausgeben, wobei die drei Signale $X_j$, $y_j$, $E_j$ durch die Ausgangssignale des ersten Teilers (505), des zweiten Teilers (506) bzw. einer Schaltung (507) zum zeitlichen Wiederausrichten gebildet werden, die das Ausgangssignal der dritten Entstapelungsberechnungsschaltung empfängt, und daß die Korrekturkoeffizienten Koeffizienten zur Korrektur mittels Extrapolation und Interpolation $\alpha$ bzw. $\gamma$ für die Entstapelungsberechnungsschaltungen sind.

9. Szintillationskamera nach Anspruch 1,
dadurch gekennzeichnet, daß die Ereignisbearbeitungsstufe (700) drei Entstapelungsberechnungsschaltungen (701, 702, 504), zwei Teiler (705, 706), eine Schaltung (507) zum zeitlichen Wiederausrichten und eine zusätzliche Berechnungsschaltung (707) enthält, wobei die zwei Teiler (705, 706) die Signale $X_{mj}$, $Y_{mj}$, bzw. $Z_{mj}$ zum Ausgeben von zwei Signale $x_{mj} = X_{mj}/Z_{mj}$ und $y_m = Y_{mj}/Z_{mj}$ empfangen, wobei die ersten und zweiten Entstapelungsberechnungsschaltungen (701, 702) die Signale $x_m$, $y_m$ empfangen und die Signale $x_j$, $y_j$ ausgeben, und die dritte Entstapelungsberechnungsschaltung (504) das Signal $E_{mj}$ empfängt und ein Signal $\hat{E}$ ausgibt, und daß die zusätzliche Berechnungsschaltung (707) einerseits das Signal $\hat{E}$ und andererseits die Korrekturkoeffizienten zum Liefern eines zusätzlichen Korrekturkoeffizienten $\Gamma$ an die ersten und zweiten Entstapelungsberechnungsschaltungen (701, 702) empfängt.

10. Szintillationskamera nach einem der Ansprüche 4 und 5,
dadurch gekennzeichnet, daß die Vervielfacher durch eine einzige mit einem Zeitmultiplexer/Demultiplexer verknüpfte Vervielfacherschaltung ersetzt sind.

11. Szintillationskamera nach einem der Ansprüche 3 bis 9,
dadurch gekennzeichnet, daß die Teiler durch eine einzige mit einem Zeitmultiplexer/Demultiplexer verknüpfte Teilerschaltung ersetzt sind.

12. Szintillationskamera nach einem der Ansprüche 1 bis 11,
dadurch gekennzeichnet, daß die Entstapelungsberechnungsschaltungen durch eine einzige dieser mit einem Zeitmultiplexer/Demultiplexer verknüpften Schaltungen ersetzt sind.

13. Szintillationskamera nach einem der Ansprüche 1 bis 12,
dadurch gekennzeichnet, daß eine Amplitudeablehnschaltung zur Verringerung der Anzahl zu bearbeitender Ereignisse vorgesehen ist.

14. Szintillationskamera nach einem der Ansprüche 1 bis 13,
dadurch gekennzeichnet, daß FIFO-Speicher stromabwärts nach den p Erfassungswegen oder stromaufwärts vor der digitalen Summierschaltung (300) vorgesehen sind.

FIG.1

**FIG.2**

FIG.3

FIG. 4

FIG. 6

FIG.5

FIG.7

EP 0 277 391 B1

FIG.8

FIG.9

24

FIG.10

FIG.11